# DEMANDE DE BREVET EUROPEEN

(11) **EP 4 427 586 A1**
(43) Date de publication de la demande: **11.09.2024**
(21) Numéro de dépôt: 24161003.9
(22) Date de dépôt: 01.03.2024
(51) Int. Cl.: A01K 43/00, G01N 33/08

(54) **NAVETTE DE SUPPORT ET D'ECLAIRAGE D'AU MOINS UN OBJET, NOTAMMENT UN OEUF AVIAIRE, ET MACHINE COMPRENANT AU MOINS UNE TELLE NAVETTE**

(30) Priorité: 09.03.2023 FR 2302214
(71) Demandeur: Unista, 85600 Treize Septiers (FR)
(72) Inventeur: BONICEL, Frédéric, 49300 CHOLET (FR); BARREAU, Frédéric, 85170 Les Lucs sur Boulogne (FR); FORGET, Anthony, 85600 Treize-Septiers (FR)
(74) Mandataire: Cabinet Chaillot

(57) **Abrégé**

L'invention concerne une navette (1) et une machine comprenant au moins une telle navette (1), laquelle navette (1) comprend : - un ensemble de maintien dont un socle (2) comporte au moins une cavité inférieure apte à recevoir une région d'extrémité inférieure d'un objet (O) et dont une bride (4) supportée par le socle (2) comporte, en regard de la cavité inférieure, une cavité supérieure apte à recevoir une région d'extrémité supérieure de l'objet (O), en utilisation, l'ensemble de maintien (2-4) étant configuré pour occuper une position de maintien en position de l'objet (O) ; - au moins un dispositif d'éclairage situé dans l'une des cavités supérieure et inférieure ; - des moyens de connexion électrique comportant un ensemble connecteur électrique (70) accessible depuis l'extérieur du socle (2) et un ensemble conducteur d'électricité formant un chemin électrique entre l'ensemble connecteur électrique (70) et le ou chaque dispositif d'éclairage.

## Description

La présente invention se rapporte de manière générale au domaine du tri d'objets.

La présente invention est particulièrement, mais non limitativement, adaptée au domaine de l'industrie avicole, et plus particulièrement, au tri d'œufs aviaires, comme par exemple des œufs de cane, selon leur sexe.

La présente invention concerne une navette de support et d'éclairage permettant d'éclairer de manière appropriée au moins un objet et de le maintenir en position correcte, et une machine, notamment une machine de tri automatique d'objets, comprenant au moins une telle navette.

La machine selon la présente invention est particulièrement adaptée à la détermination in ovo, de manière non invasive, du genre mâle ou femelle d'un embryon contenu dans un œuf fécondé, et au tri des œufs selon leur genre. Un tel tri est nécessaire pour certains types de production qui impliquent de ne conserver que les individus mâles ou femelles produits. Par exemple, pour des questions de qualité, la production de foie gras se fait exclusivement par gavage des canards mâles, alors que pour la production d'œufs de poule, seules les femelles sont conservées.

Toutefois, cette machine pourra également être mise en œuvre pour l'inspection et le tri de tous autres objets pouvant être triés selon l'une de leurs caractéristiques observables.

Dans le domaine avicole, on connaît depuis de nombreuses années la problématique récurrente de la détermination du sexe des œufs avant leur éclosion. En effet, la détermination du genre des embryons à un stade précoce de l'incubation permet d'améliorer la production avicole, avec à la fois un gain de temps et un gain financier. Par conséquent, un certain nombre de techniques de détermination ont été développées afin d'évaluer le sexe d'un œuf d'oiseau non éclot.

Une technique connue est basée sur la mesure de la quantité de lumière transmise par un œuf. Cette technique de mirage est largement utilisée et est, par exemple, décrite dans le brevet américain US 5 745 228 A. Ce brevet américain décrit un appareil comprenant un casier à œufs disposé horizontalement sur un dispositif de convoyage et configuré pour porter plusieurs rangées d'œufs, au moins une source de lumière positionnée sur un côté du casier et au moins un détecteur de lumière positionné sur l'autre côté du casier, en regard de l'au moins une source de lumière et dans un même plan vertical, le dispositif de convoyage amenant le casier à œufs à défiler entre l'au moins une source de lumière et l'au moins un détecteur.

On peut également citer le brevet français FR 2 935 051 B1 qui divulgue un dispositif de mirage automatique d'œufs analogue. Ce dispositif comprend des plateaux d'incubation d'œufs comportant des alvéoles dans lesquelles sont disposés des œufs à mirer, des moyens de convoyage pour transporter les plateaux selon un plan de convoyage, un support, disposé au-dessus des moyens de convoyage et portant de manière fixe des moyens d'éclairement et des moyens de capture d'image, lequel support est apte à être déplacé verticalement entre une position relevée et une position de mirage, dans laquelle il vient en appui sur la partie supérieure des œufs.

Cependant, dans ces appareils et dispositifs de l'état antérieur de la technique, les œufs sont positionnés dans un casier à œufs ou un plateau d'incubation dans lequel les œufs peuvent prendre une position quelconque, laquelle position dépend des contacts entre les œufs et les parois du casier ou du plateau. Or, le positionnement correct et stable des œufs revêt une importance particulière pour garantir un examen fiable des œufs.

De plus, un tel appareil ou dispositif prévoit une ou des sources de lumière sur un seul côté de l'objet à mirer, ce qui peut s'avérer insuffisant.

Ainsi, ces appareils et dispositifs de l'état antérieur de la technique ne permettent pas d'obtenir des conditions de présentation optimales, notamment en termes de positionnement et d'éclairage du ou des objets, permettant un examen rapide et fiable du ou des objets présentés.

Il existe donc un besoin pour des moyens permettant d'optimiser les conditions aussi bien d'éclairage que de positionnement et de maintien du ou des objets à trier, tout en s'adaptant aux dimensions des objets et aux écarts dimensionnels entre divers objets, sans risque d'endommagement des objets manipulés et pouvant être mis en œuvre facilement à une échelle industrielle.

La présente invention vise à résoudre les inconvénients de l'état antérieur de la technique, en proposant une navette intégrant à la fois des moyens de support d'au moins un objet à trier et des moyens d'éclairage de celui-ci, les moyens de support et d'éclairage étant configurés pour une présentation optimale de l'au moins un objet. La présente invention propose également une machine, notamment une machine de tri, comprenant au moins une telle navette, laquelle machine est simple, fiable, économique et facilement mise en œuvre à l'échelle industrielle. Dans un mode de réalisation particulier de l'invention, dans lequel la machine est adaptée au tri d'œufs, les inconvénients de l'état antérieur de la technique sont en outre palliés par la disposition d'au moins un plateau d'orientation et d'alignement d'œufs en amont de l'au moins une navette.

La présente invention a donc pour objet une navette de support et d'éclairage d'au moins un objet, notamment un objet à trier tel qu'un œuf aviaire, destinée à être positionnée ou déplacée dans un poste de visiométrie ou d'inspection optique, laquelle navette comporte un ensemble de maintien configuré pour supporter et maintenir en position l'au moins un objet, et un système d'éclairage configuré pour éclairer l'au moins un objet, la navette étant caractérisée par le fait que :
- l'ensemble de maintien comprend un socle et une bride supportée par le socle, le socle comporte au moins un siège définissant une cavité inférieure agencée pour recevoir une région d'extrémité inférieure d'un objet et ayant une ouverture de réception inférieure apte à coopérer avec l'objet, la bride comporte, en regard du ou de chaque siège, une cavité supérieure agencée pour recevoir une région d'extrémité supérieure de l'objet et ayant une ouverture de réception supérieure apte à coopérer avec l'objet, l'ouverture de réception inférieure et l'ouverture de réception supérieure associée étant orientées l'une vers l'autre et selon un même axe, en utilisation, l'ensemble de maintien étant configuré pour être apte à occuper une position écartée, dans laquelle la ou chaque ouverture de réception inférieure et l'ouverture de réception supérieure associée sont espacées l'une de l'autre d'une distance autorisant le dépôt d'un objet sur le siège et son retrait, et une position de maintien, dans laquelle la ou chaque ouverture de réception inférieure et l'ouverture de réception supérieure associée sont rapprochées l'une de l'autre d'une distance permettant le maintien et l'immobilisation d'un objet entre le socle et la bride, avec les régions d'extrémité inférieure et supérieure de l'objet reçues dans la cavité inférieure et dans la cavité supérieure, respectivement ;
- le système d'éclairage comprend, dans au moins l'une des cavités inférieure et supérieure, un dispositif d'éclairage configuré pour émettre de la lumière, par exemple un faisceau lumineux, en direction de l'ouverture de réception associée ; et
- la navette comprenant en outre des moyens de connexion électrique comportant un ensemble connecteur électrique accessible depuis l'extérieur du socle et un ensemble conducteur d'électricité formant un chemin électrique entre l'ensemble connecteur électrique et le ou chaque dispositif d'éclairage, de telle sorte qu'en utilisation, lorsque l'ensemble connecteur est relié à des moyens d'injection courant, le ou chaque dispositif d'éclairage est apte à être alimenté en électricité.

La navette selon la présente invention permet une présentation optimale d'au moins un objet, laquelle présentation optimale implique un maintien correct et fiable de l'au moins un objet et son éclairage approprié.

En effet, un objet supporté par la navette est maintenu à la fois par le dessus et par le dessous lorsque l'ensemble de maintien est en position de maintien. En l'absence de la bride et donc de moyens de maintien par le dessus, le maintien en position fixe de l'objet ne serait pas assuré, et l'objet risquerait de chuter, en particulier lors du déplacement de la navette.

Le fait que la cavités inférieure et supérieure soient agencées, notamment dimensionnées, pour recevoir uniquement une région d'extrémité inférieure et une région d'extrémité supérieure, respectivement, de l'objet permet au reste de l'objet de ne pas être masqué. Ainsi, dans le cas où l'axe de l'objet est positionné verticalement, l'objet peut être vu et inspecté sur 360 degrés autour de son axe.

La présence d'un dispositif d'éclairage supérieur et/ou d'un dispositif d'éclairage inférieur permet d'éclairer l'objet par le dessus ou par le dessous, ou les deux.

Par ailleurs, de tels moyens de connexion électrique, notamment comprenant un ensemble connecteur électrique accessible depuis l'extérieur du socle, autrement dit solidaire du socle, permettent d'alimenter le ou les dispositifs d'éclairage de manière sans fil, et donc de ne pas gêner les déplacements de la navette. De plus, ces moyens de connexion permettent d'obtenir une navette légère, contrairement à une navette qui comprendrait une batterie pour alimenter son système d'éclairage.

De préférence, la bride est montée mobile à coulissement libre vertical par rapport au socle, le socle portant au moins un guide vertical le long duquel la bride est apte à coulisser. Ainsi, le déplacement de l'ensemble de maintien entre les positions écartée et de maintien est provoqué par le déplacement en translation verticale de la bride portant la ou les cavités supérieures par rapport au socle portant la ou les cavités inférieures correspondantes. Un tel montage coulissant de la bride permet de s'adapter à des objets de diverses hauteurs.

De préférence, l'au moins un guide vertical comprend deux montants verticaux parallèles.

De préférence, l'ensemble connecteur électrique fait saillie depuis une face du socle agencée parallèlement au plan de déplacement en translation de la bride.

Avantageusement, la bride porte au moins un aimant agencé côté sa face inférieure dirigée vers le socle, de préférence, un aimant disposé au voisinage du ou de chaque guide vertical. Ainsi, la bride est apte à être déplacée facilement le long de l'au moins un guide, par un organe de manipulation venant se placer sous la bride et coopérant avec les aimants, grâce à la force des aimants. De tels aimants permettent de s'affranchir du montage de ressorts sur les guides afin de solliciter la bride en direction du socle.

De préférence, pour le ou chaque dispositif d'éclairage supérieur, le chemin électrique comprend des fils électriques intégrés au socle et reliant l'ensemble connecteur électrique à l'extrémité inférieure du ou de chaque guide vertical, le ou chaque guide vertical réalisé en un matériau électroconducteur et une pluralité d'éléments conducteurs intégrés à la bride et reliant le ou de chaque guide vertical et le dispositif d'éclairage supérieur, et, pour le ou chaque dispositif d'éclairage inférieur, le chemin électrique comprend des fils électriques intégrés au socle et reliant directement l'ensemble connecteur électrique et le dispositif d'éclairage inférieur.

Le fait d'avoir un chemin électrique entre le socle et la bride formé par au moins un guide vertical en matériau électroconducteur plutôt que par des fils électriques traversant le ou chaque guide permet d'éviter qu'un élément environnant se déplaçant au voisinage de la navette ne se prenne dans un fil et ne l'arrache. Un tel chemin électrique est donc plus fiable.

De préférence, le ou chaque guide vertical est réalisé en acier inoxydable.

Dans un mode de réalisation particulier, la pluralité d'éléments conducteurs intégrés à la bride comprend, pour le ou chaque guide vertical : un palier en bronze monté autour du guide, une bride reliant un ressort de traction au palier, une cosse reliée à la bride, et une vis assurant le contact entre le dispositif d'éclairage et le ressort.

Avantageusement, le système d'éclairage comprend un dispositif d'éclairage supérieur dans la ou chaque cavité supérieure et un dispositif d'éclairage inférieur dans la ou chaque cavité inférieure, chaque dispositif d'éclairage supérieur et le dispositif d'éclairage inférieur associé étant avantageusement agencés pour émettre des faisceaux lumineux passant par l'axe commun des ouvertures de réception associées. Ainsi, dans le cas où l'objet est positionné de telle sorte que son axe est vertical, celui-ci est éclairé par des faisceaux lumineux passant par son axe, autrement dit, l'objet est traversé par les faisceaux lumineux.

De préférence, chaque dispositif d'éclairage comprend une source lumineuse à semi-conducteurs, telle qu'une diode électroluminescente, une lentille et un verre de protection délimitant dans la cavité un espace de réception de dispositif d'éclairage et un espace de réception d'objet.

Dans le cas où la navette selon la présente invention est appliquée à l'industrie avicole, notamment au tri d'œufs selon leur genre, il est préférable que la navette comprennent un unique siège, et donc une seule cavité inférieure et une seule cavité supérieure.

Avantageusement, les cavités supérieure et inférieure sont configurées de telle sorte que leurs ouvertures épousent la forme de l'objet à maintenir, autrement dit viennent en contact étroit avec l'objet.

Ainsi, dans le cas où l'objet est un œuf, chaque cavité a, de préférence, une forme en tulipe, en entonnoir, sensiblement tronconique ou ogivale allant en s'épanouissant vers l'ouverture de réception. Une telle forme permet à la fois un bon maintien de l'œuf et une étanchéité lumineuse.

La présente invention a également pour objet une machine, notamment machine de tri automatique d'objets, ladite machine étant caractérisée par le fait qu'elle comprend au moins une navette de support et d'éclairage telle que définie ci-dessus, la machine comprenant en outre un poste de réception d'objets, un poste de visiométrie et un poste de récupération d'objets, des moyens de transport pour déplacer l'au moins une navette selon un trajet passant par les divers postes, un ensemble de préhension amont apte à saisir au moins un objet au poste de réception et à le positionner dans l'au moins une navette, et un ensemble de préhension aval apte à saisir au moins un objet dans l'au moins une navette et à le transférer au poste de récupération, le poste de visiométrie comprenant une pluralité de caméras de prise de vues associées à des moyens d'analyse automatique et des moyens d'injection de courant aptes à être connectés à l'ensemble connecteur électrique de l'au moins une navette.

Grâce à la au moins une navette selon la présente invention, le ou les objets sont présentés au niveau du poste de visiométrie en étant mis en scène de manière optimale.

De préférence, la machine comprend une pluralité de navettes supportant et éclairant chacune un seul objet. Les navettes sont positionnées les unes à la suite des autres dans un certain ordre, dit ordre de départ, au poste de réception d'objets, peuvent être déplacées les unes à la suite des autres en conservant l'ordre de départ jusqu'au poste de visiométrie, puis être déplacées à nouveau les unes à la suite des autres jusqu'au poste de récupération où elles sont à nouveau positionnées les unes à la suite des autres, mais éventuellement rangées dans un ordre différent de l'ordre de départ en fonction du résultat de l'analyse réalisée par les moyens d'analyse automatique.

De préférence, chaque ensemble de préhension comporte une pluralité d'outil de saisie, chaque outil de saisie comprenant une ventouse adaptée à la préhension d'un objet.

De préférence, chaque ensemble de préhension comporte également un outil de manipulation de bride. Un tel outil de manipulation de bride peut présenter la forme d'un râteau comprenant une pluralité de paires de doigts, chaque paire de doigts étant apte à venir se placer sous une bride, le cas échéant au niveau des aimants, et à déplacer la bride vers le haut et vers le bas le long du ou des guides verticaux. En variante, un tel outil de manipulation de bride peut comprendre au moins une pince mobile apte à être positionnée verticalement au-dessus d'une bride et à se fermer sous la bride afin de permettre son déplacement le long des guides verticaux. Nous soulignons que tout outil apte à venir se placer sous une bride ou apte à saisir un bride et à la déplacer pourrait convenir.

De préférence, la pluralité de caméras de prise de vues comprend une première paire de caméras disposée d'un côté du trajet de la navette et une seconde paire de caméras disposée de l'autre côté du trajet de la navette.

De préférence, une fois les moyens d'injection de courant accouplés à l'ensemble connecteur électrique d'une navette, dans le cas où la navette comprend un dispositif d'éclairage supérieur et un dispositif d'éclairage inférieur, chaque dispositif d'éclairage est commandé de manière successive. Une telle commande indépendante des divers dispositifs d'éclairage permet d'obtenir des images de l'objet éclairé uniquement par le dessus et des images de l'objet éclairé uniquement par le dessous. De plus, l'intensité d'éclairage de chaque dispositif d'éclairage peut être réglée, notamment en fonction de l'opacité de l'objet.

Dans un mode de réalisation particulier, les moyens de transport comprennent un moteur plan à sustentation magnétique sur lequel la ou les navettes sont disposées. Un tel moteur plan permet d'obtenir un navette automotrice apte à se déplacer automatiquement d'un poste à un autre.

Le moteur plan pourrait être remplacé par un moteur linéaire, un actionneur électromagnétique, etc.

En variante, les moyens de transport pourraient comprendre au moins un convoyeur sur lequel l'au moins une navette est placée.

Dans un mode de réalisation préféré, la machine est adaptée au tri d'œufs, le poste de réception comprenant au moins un plateau d'orientation et d'alignement d'œufs, le ou chaque plateau comprenant au moins un module d'orientation et d'alignement, le ou chaque module comprenant deux rouleaux rotatifs axialement parallèles et espacés l'un de l'autre d'une distance permettant le positionnement d'un œuf sur et entre les deux rouleaux, et un mécanisme d'entraînement en rotation configuré pour entraîner les deux rouleaux d'un même module en contre-rotation l'un par rapport à l'autre et vers l'espace inter-rouleaux. Ainsi, pour le ou chaque module, la mise en contre-rotation des rouleaux permet d'orienter correctement les œufs positionnés sur le module avec leur axe longitudinal à l'horizontale.

La présence d'un tel plateau d'orientation et d'alignement d'œufs en amont du poste de visiométrie permet donc à l'ensemble de préhension amont de saisir les œufs dans de bonnes conditions, à savoir de manière bien orientée, afin de pouvoir les positionner correctement sur la navette, à savoir avec longitudinal vertical et le petit bout vers le haut. En effet, les œufs reçus dans les casiers ne sont pas calibrés et présentent donc diverses dimensions en longueur (variations de l'ordre de 19 mm), en largeur, divers poids et divers diamètres (variations de l'ordre de 10 mm) qui ne favorisent pas un rangement et un positionnement correct des œufs dans le casier. Une réorientation des œufs avant leur positionnement dans les navettes est donc nécessaire.

Dans un mode de réalisation particulier, le mécanisme d'entraînement en rotation comprend une crémaillère, pour le ou chaque module, un premier pignon en prise avec la crémaillère et solidaire de l'un des deux rouleaux, un pignon intermédiaire solidaire du rouleau portant le premier pignon et s'engrenant avec un second pignon solidaire de l'autre rouleau.

De préférence, les rouleaux sont réalisés en un matériau lisse, tel que de l'acier inoxydable. Un tel matériau lisse permet aux œufs de glisser facilement sur les rouleaux.

Avantageusement, chaque rouleau comprend au moins une section de calage, la ou chaque section de calage comprenant une gorge cylindrique agencée entre deux portions tronconiques tournées l'une vers l'autre, les sections de calage associées de deux rouleaux d'un même module étant alignées radialement. Une telle forme permet à l'objet de se caler dans les gorges associées et donc de s'aligner dans le sens de la longueur des rouleaux quelle que soit sa taille.

De préférence, chaque module a trois paires de sections de calage.

Avantageusement, chaque module comporte, de part et d'autre de la ou chaque paire de sections de calage, au niveau des gorges, un guide extérieur faisant saillie au-dessus des gorges. De tels guides extérieurs permettent d'assurer le recentrage de l'objet selon l'axe longitudinal des rouleaux.

De préférence, le ou chaque plateau comprend une pluralité de modules d'orientation et d'alignement dont les rouleaux sont agencés parallèlement les uns aux autres, les modules sont montés mobiles en translation le long de deux rails de guidage parallèles entre eux et dont les axes perpendiculaires aux axes des rouleaux, par l'intermédiaire de patins montés coulissants le long des rails, chaque module étant supporté par deux patins solidaires de chaque extrémité longitudinale du module. Ainsi, les modules sont aptes à être déplacés selon une direction transversale à la direction longitudinale des rouleaux. Ils peuvent donc être rapprochés ou écartés les uns des autres.

En variante, chaque module pourrait être monté fixe sur un support avec un espacement prédéterminé entre les modules. Dans le cas où l'ensemble de préhension amont comprend une pluralité d'outils de saisie espacés les uns des autres d'un certain pas, l'espacement entre les modules correspondant au pas des outils de saisie.

Chaque plateau peut comprendre huit modules, soit seize rouleaux.

Dans un mode de réalisation particulier, le ou chaque plateau comprend un système de mise au pas des modules configuré pour piloter le déplacement en translation de la pluralité de modules le long des rails de guidage avec un pas constant entre les modules, lequel système de mise au pas comprend une pluralité de bielles articulées entre elles autour d'axes perpendiculaires au plan de déplacement en translation des modules, chaque bielle étant solidaire d'un module, l'axe d'articulation entre deux bielles dites bielles centrales étant reçu dans un moyen de guidage en translation suivant une direction de translation perpendiculaire à l'axe d'articulation et parallèle aux axes des rouleaux, deux vérins d'actionnement disposés de part et d'autre de la pluralité de bielles, parallèlement entre eux et orthogonalement aux axes des rouleaux et aux axes d'articulation, étant solidaires de deux modules, de telle sorte qu'un déplacement en translation relatif entre les deux modules d'un pas prédéterminé, sous l'effet des vérins, provoque un déplacement en translation synchrone de chaque module avec un pas entre modules égal audit pas prédéterminé.

L'absence de motorisation sur le plateau permet un démontage et un entretien aisés du plateau.

Avantageusement, le ou chaque plateau est monté mobile en translation le long de deux rails transversaux de guidage, les rails transversaux étant parallèles entre eux et parallèles aux axes des rouleaux, chaque extrémité longitudinale du ou de chaque plateau étant solidaire d'un support latéral monté coulissant le long d'un rail respectif, l'un des deux support du ou de chaque plateau étant solidaire d'une courroie synchrone tendue entre deux poulies, l'une des poulies étant accouplée à un motoréducteur. Ainsi, un seul moteur permet le déplacement en translation d'un ou de plusieurs plateaux.

De préférence, la machine comprend deux plateaux dont un plateau inférieur et un plateau supérieur, l'un des supports latéraux du plateau supérieur étant solidaire du brin supérieur de la courroie, le support latéral correspondant du plateau inférieur étant solidaire du brin inférieur de la courroie, de telle sorte que les plateaux supérieurs et inférieurs peuvent être positionnés de manière décalée horizontalement l'un par rapport à l'autre.

Le plateau d'orientation et d'alignement d'œufs tel que décrit ci-dessus peut être utilisé indépendamment des autres caractéristiques de la machine décrite ci-dessus. Ainsi, est divulgué par la présente un plateau d'orientation et d'alignement d'œufs comprenant au moins un module d'orientation et d'alignement, le ou chaque module comprenant deux rouleaux rotatifs axialement parallèles et espacés l'un de l'autre d'une distance permettant le positionnement d'un œuf sur et entre les deux rouleaux, et un mécanisme d'entraînement en rotation configuré pour entraîner les deux rouleaux d'un même module en contre-rotation l'un par rapport à l'autre et vers l'espace inter-rouleaux. Ce plateau peut présenter les mêmes caractéristiques que celles décrites ci-dessus en liaison avec la machine selon la présente invention.

Pour mieux illustrer l'objet de la présente invention, on va en décrire ci-après, à titre illustratif et non limitatif, un mode de réalisation particulier, avec référence au dessin annexé.

Sur ce dessin :
[Fig. 1] est une vue en perspective de la navette selon un mode de réalisation particulier de la présente invention, appliquée au support et à l'éclairage d'un œuf ;
[Fig. 2] est une vue de côté, en coupe verticale, de la navette de la Figure 1 ;
[Fig. 3] est une vue en perspective de dessus du socle seul, montrant le dispositif d'éclairage inférieur ;
[Fig. 4] est une vue de dessous du socle, montrant les moyens de connexion électrique intégrés au socle ;
[Fig. 5] est une vue de dessus de la bride, montrant l'intérieur de la bride ;
[Fig. 6] est une vue en perspective de dessous de la bride seule, montrant le dispositif d'éclairage supérieur ;
[Fig. 7] est une vue en perspective au niveau du poste de visiométrie de la machine selon un mode de réalisation particulier de la présente invention, montrant une navette connectée aux moyens d'injection de courant ;
[Fig. 8] est une vue de dessus au niveau du poste de visiométrie de la Figure 7, montrant la navette arrêtée devant une pluralité de caméras de prise de vues ;
[Fig. 9] est une vue en perspective d'un module d'orientation et d'alignement d'une machine selon un mode de réalisation particulier de la présente invention, adaptée au tri d'œufs ;
[Fig. 10] est une vue de côté du module de la Figure 9 ;
[Fig. 11] est une vue de dessus d'un plateau d'orientation et d'alignement d'œufs comprenant une pluralité de modules ;
[Fig. 12] est une vue en perspective, agrandie au niveau de deux modules, du plateau de la Figure 11 ;
[Fig. 13] est une vue de dessous du plateau de la Figure 11 ;
[Fig. 14] est une vue en perspective de dessus d'un système comprenant deux plateaux d'orientation et d'alignement d'œufs mobiles en translation ; et
[Fig. 15] est une vue de côté, depuis le côté courroie, du système à deux plateaux de la Figure 14.

Dans la description qui va suivre, les termes « supérieur », « inférieur », « au-dessus » et « au-dessous » s'entendent dans la direction verticale, autrement dit dans une direction perpendiculaire au plan horizontal sur lequel la navette 1 selon l'invention repose.

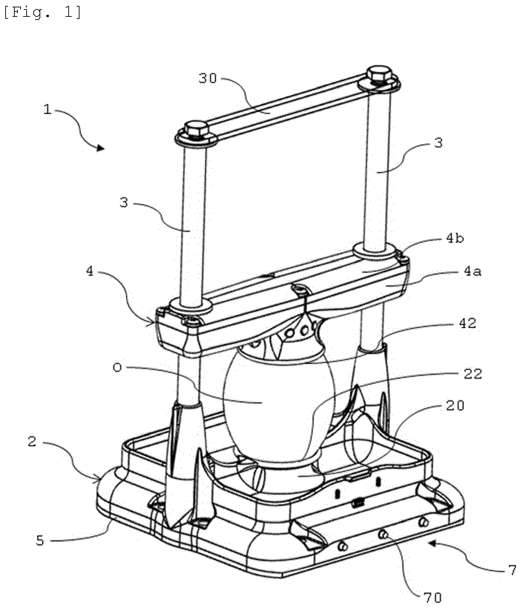

Si l'on se réfère tout d'abord aux Figures 1 à 6, on peut voir que la navette 1 selon un mode de réalisation préféré de la présente invention comprend un ensemble de maintien, un système d'éclairage 6 et des moyens de connexion électrique 7.

Dans le mode de réalisation représenté, l'ensemble de maintien est configuré pour supporter et maintenir en position un œuf O. Il convient toutefois de souligner que l'ensemble de maintien pourrait être configuré pour supporter et maintenir en position plusieurs œufs O ou un nombre quelconque d'objets autres que des œufs O.

Dans le mode de réalisation représenté, l'ensemble de maintien comprend essentiellement un socle 2, deux montant verticaux 3 et une bride 4 montée mobile par rapport au socle 2.

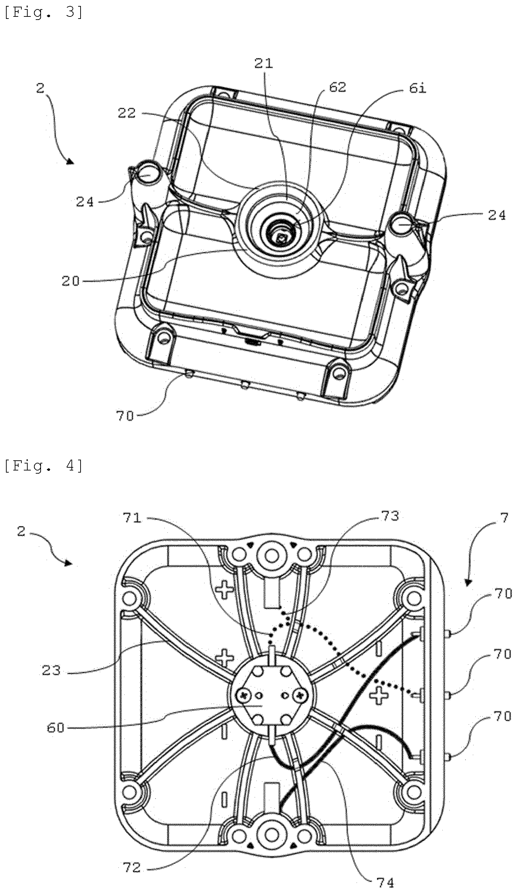

Comme on peut le voir sur les Figures 3 et 4, le socle 2 comprend un corps présentant deux faces opposées supérieure et inférieure et quatre faces latérales opposées deux à deux. Un lest 5 d'équilibrage est avantageusement fixé à la face inférieure afin d'abaisser le centre de gravité de la navette 1 et d'augmenter sa stabilité. Ce lest 5 se présente sous la forme d'une plaque plane recouvrant la face inférieure. Le lest 5 peut être ajouré.

Le corps du socle 2 comprend un siège 20 configuré pour intégrer un dispositif d'éclairage inférieur 6i et recevoir une région d'extrémité inférieure de l'œuf O. Le siège 20 s'étend ici de la face inférieure à la face supérieure et fait saillie au-dessus de la face supérieure. Le siège 20 est situé au centre du socle 2. Autrement dit, l'axe longitudinal du siège 20 coïncide avec l'axe central vertical du socle 2. Le siège 20 comporte une cavité 21, dite cavité inférieure 21, ouverte par le dessus et par le dessous. L'ouverture par le dessous, au niveau de la face inférieure, est une ouverture par exemple de forme hexagonale. L'ouverture par le dessus 22 est configurée pour coopérer avec une région d'extrémité inférieure de l'œuf O et est donc désignée ici par ouverture de réception inférieure 22. L'ouverture de réception inférieure 22 a une forme circulaire.

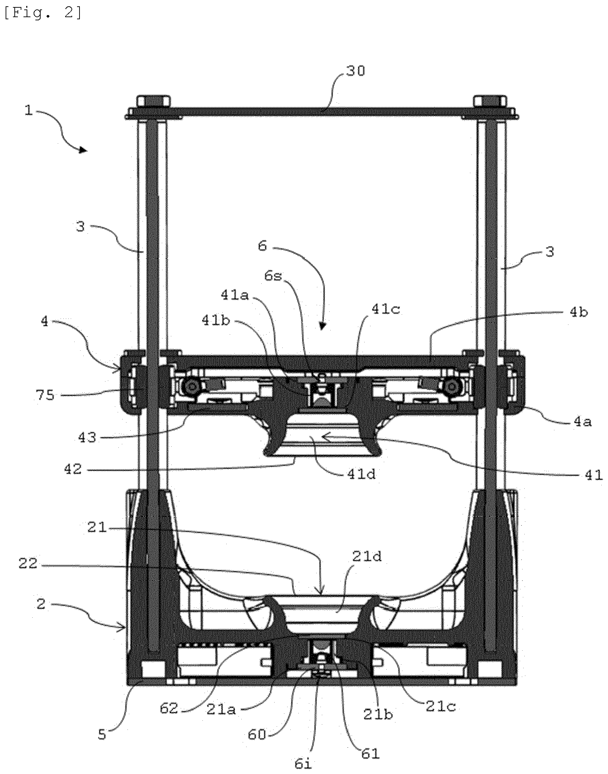

Comme on peut le voir sur la Figure 2, la cavité inférieure 21 est formée d'une pluralité de sous-cavités 21a-21d, en particulier de quatre sous-cavités. La pluralité de sous-cavités 21a-21d comprend une sous-cavité de section hexagonale 21a s'étendant verticalement depuis l'ouverture par le dessous, une première sous-cavité de section circulaire 21b ayant un premier diamètre et une seconde sous-cavité de section circulaire 21c ayant un second diamètre supérieur au premier diamètre, et une sous-cavité de réception d'œuf 21d ayant une forme allant en s'épanouissant vers l'ouverture de réception inférieure 22, notamment une forme de tulipe, d'entonnoir, sensiblement tronconique ou ogivale. La forme de la sous-cavité de réception d'œuf 21d est ainsi adaptée à la réception d'un œuf O par l'un de ses bouts, de manière analogue à la coupelle d'un coquetier. Un espace intérieur est délimité par les faces supérieure, inférieure et latérales et par la partie du siège 20 située au-dessous de la face supérieure. Des parties de renforcement 23 s'étendent dans l'espace intérieur, entre le siège 20 et les faces latérales. De part et d'autre du siège 20, au niveau du centre de deux faces latérales opposées, le socle 2 comprend deux logements verticaux 24 formés d'un seul tenant avec le corps. Les deux logements verticaux 24 sont parallèles entre eux et s'étendent selon un axe longitudinal vertical, donc parallèlement à l'axe longitudinal du siège 20. Les logements 24 sont configurés pour recevoir les deux montants verticaux 3.

Les deux montants verticaux 3 sont des montants cylindriques reçus dans les logements 24 de section circulaire correspondante. Chaque montant 3 fait saillie au-dessus du socle 2 sur une certaine hauteur. Les deux montants 3 se situent dans un même plan vertical passant par un plan de symétrie du siège 20. Chaque montant 3 est réalisé en un matériau électroconducteur, notamment en acier inoxydable. Les deux montants 3 peuvent être reliés entre eux, au niveau de leur extrémité supérieure, par une barre d'écartement 30. La barre d'écartement 30 peut être solidarisée à chaque extrémité supérieure par vissage.

La bride mobile 4 est montée à coulissement libre vertical le long des deux montants verticaux 3. A cet effet, la bride 4 est traversée par deux orifices verticaux 40. Chaque orifice 40 est configuré pour être traversé par un montant 3 et pour glisser le long de ce montant 3.

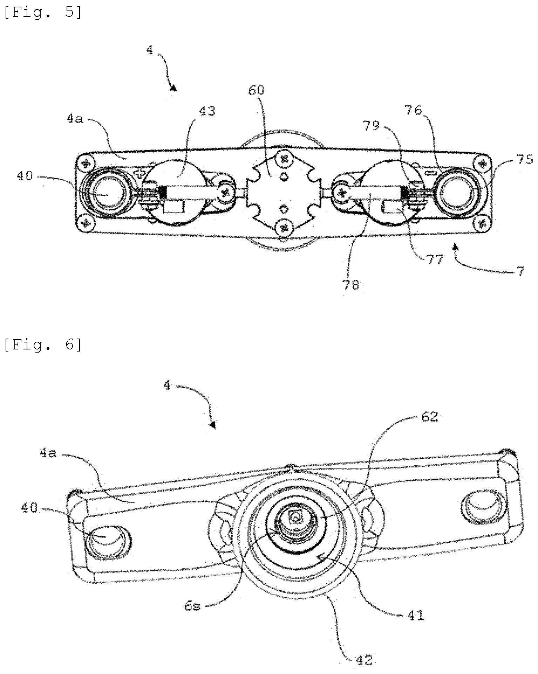

La bride 4 comprend un corps en deux parties assemblées entre elles par vissage, à savoir un boîtier 4a et un couvercle 4b recouvrant et fermant le boîtier 4a. Comme on peut le voir sur les Figures 5 et 6, le boîtier 4a présente deux faces opposées supérieure et inférieure et quatre faces latérales opposées deux à deux. La face supérieure est une face plane de forme rectangulaire s'étendant dans un plan horizontal, donc orthogonalement aux axes des montants 3. La face inférieure est sensiblement plane, mais pourrait présenter une forme particulière, notamment une forme apte à coopérer avec des outils de manipulation de forme complémentaire. Le boîtier 4a comprend, en regard du siège 20, une cavité dite cavité supérieure 41 configurée pour intégrer un dispositif d'éclairage supérieur 6s et recevoir une région d'extrémité supérieure de l'œuf O. La cavité supérieure 41 s'étend ici de la face supérieure du boîtier 4a à la face inférieure et fait saillie au-dessous de la face inférieure. La cavité supérieure 41 s'étend selon un axe longitudinal situé au centre de la bride 4, autrement dit à égale distance de chaque montant 3. L'axe longitudinal de la cavité supérieure 41 est un axe vertical qui coïncide avec l'axe longitudinal de la cavité inférieure 21. De manière analogue à la cavité inférieure 21, la cavité supérieure 41 est ouverte par le dessus et par le dessous. L'ouverture par le dessus, au niveau de la face supérieure, est une ouverture par exemple de forme hexagonale. L'ouverture par le dessous 42 est configurée pour coopérer avec une région d'extrémité supérieure de l'œuf O et est donc appelée ouverture de réception supérieure 42. L'ouverture de réception supérieure 42 a une forme circulaire.

Comme on peut le voir sur la Figure 2, la cavité supérieure 41 est formée d'une pluralité de sous-cavités 41a-41d, en particulier de quatre sous-cavités. La pluralité de sous-cavités 41a-41d comprend une sous-cavité de section hexagonale 41a s'étendant verticalement depuis l'ouverture par le dessus, une première sous-cavité de section circulaire 41b ayant un premier diamètre et une seconde sous-cavité de section circulaire 41c ayant un second diamètre supérieur au premier diamètre, et une sous-cavité de réception d'œuf 41d ayant une forme allant en s'épanouissant vers l'ouverture de réception supérieure 42, notamment une forme de tulipe, d'entonnoir, sensiblement tronconique ou ogivale. La forme de la sous-cavité de réception d'œuf 41d est ainsi adaptée à la réception d'un œuf O par l'un de ses bouts.

Comme on peut le voir sur la Figure 5, deux espaces intérieurs, communiquant avec la cavité supérieure 41, sont délimités entre les faces supérieure, inférieure et latérales. Comme on peut le voir sur les Figures 2 et 5, dans chacun des deux espaces intérieurs peut être reçu un aimant 43, notamment un aimant circulaire. Un aimant 43 pourra donc être prévu, de chaque côté de la cavité supérieure 41, au voisinage des montants 3. Chaque aimant 43 est positionné en contact avec la face inférieure.

En utilisation, afin de pouvoir positionner un œuf O sur le siège 20, la bride 4 mobile est tout d'abord amenée à occuper une position écartée, par tout moyen de manipulation approprié. De tels moyens de manipulation peuvent comprendre deux doigts aptes à venir se placer au-dessous de la bride 4, le cas échéant au niveau des deux aimants 43, et à soulever la bride 4, autrement dit à la déplacer en translation verticale le long des deux montants 3 à l'opposé du socle 2. Dans cette position écartée, la distance entre l'ouverture de réception inférieure 22 et l'ouverture de réception supérieure 42 est plus grande que la hauteur de l'œuf O à placer. Puis l'œuf O est positionné sur le siège 20, avec l'axe longitudinal de l'œuf O selon la verticale et le gros bout de l'œuf O reçu dans la cavité inférieure 21, en particulier dans la sous-cavité de réception d'œuf 21d, l'ouverture de réception inférieure 22 venant en contact direct avec la coquille de l'œuf O. Une fois l'œuf O ainsi supporté par le socle 2, la bride 4 mobile est amenée à occuper une position de maintien. Pour cela, la bride 4 est amenée à coulisser le long des deux montants 3, en direction du socle 2, jusqu'à ce que l'ouverture de réception supérieure 42 soit en contact direct avec la coquille de l'œuf O. Dans cette position de maintien, comme on peut le voir sur la Figure 1, le petit bout de l'œuf O est reçu dans la cavité supérieure 41, en particulier dans la sous-cavité de réception d'œuf 41d, et l'œuf O se trouve bloqué en position fixe entre la bride 4 et le socle 2, en particulier entre les ouvertures de réception supérieure 42 et inférieure 22. A l'exception du petit bout et du gros bout de l'œuf O reçus dans les cavités 21, 41, tout le reste de l'œuf O est laissé libre sur 360 degrés autour de l'axe longitudinal de l'œuf O. Autrement dit, le reste de l'œuf O ne vient en contact avec aucune paroi et demeure visible.

Bien que dans le mode de réalisation préféré la bride 4 est montée mobile par rapport au socle 2, il convient de souligner que l'ensemble de maintien n'est pas limité à cette configuration. En particulier, il serait possible de prévoir une configuration dans laquelle le siège 20 serait monté mobile par rapport à la bride 4. De même, le guidage du mouvement de coulissement de la bride 4 n'est pas limité aux deux montants verticaux 3 et pourrait être réalisé par tout autre moyen de guidage vertical.

On pourrait également prévoir un agencement dans lequel le déplacement relatif entre le socle 2 et la bride 4 n'est pas une simple translation, comme décrit ci-dessus, mais un autre mouvement simple, comme une rotation autour d'un axe vertical ou horizontal, ou un mouvement composé, comme une combinaison d'une rotation et d'une translation.

L'ensemble de maintien porte le système d'éclairage 6. Dans le mode de réalisation représenté, le système d'éclairage 6 est configuré pour éclairer un œuf O à la fois depuis le dessus de l'œuf O et depuis le dessous de l'œuf O. Il convient toutefois de souligner que le système d'éclairage 6 pourrait être configuré pour éclairer une pluralité d'objets et/ou configuré pour éclairer le ou les objets soit depuis le dessus, soit depuis le dessous.

Dans le mode de réalisation représenté, le système d'éclairage 6 comprend un dispositif d'éclairage supérieur 6s logé dans la cavité supérieure 41 et un dispositif d'éclairage inférieur 6i logé dans la cavité inférieure 21.

Comme on peut le voir sur la Figure 2, chaque dispositif d'éclairage 6i, 6s comprend une diode électroluminescente 60, une lentille tubulaire 61 en appui contre la diode 60 et un verre de protection 62 recouvrant la lentille 61. Comme on peut le voir sur les Figures 4 et 5, la diode électroluminescente 60 est plate et de forme ici sensiblement hexagonale complémentaire de la sous-cavité de section hexagonale 21a, 41a dans laquelle elle est reçue et fixée, notamment par vissage. Ainsi, la diode électroluminescente 60 ferme l'ouverture de la cavité 61, 41 opposée à l'ouverture de réception 22, 42. Comme on peut le voir sur les Figures 3 et 6, la lentille 61 est dimensionnée pour être reçue dans la première sous-cavité de section circulaire 21b, 41b. Le verre de protection 62 est circulaire et dimensionné pour être reçu dans la seconde sous-cavité de section circulaire 21c, 41c. Le verre 62 est fixé par collage à la paroi de la sous-cavité. Ce verre de protection 62 sépare ainsi la cavité 21, 41 en un espace de réception du dispositif d'éclairage 6i, 6s et en un espace de réception de l'œuf O. Ce verre 62 permet de protéger la diode électroluminescente 60 et la lentille 61 vis-à-vis de salissures provenant de l'œuf O et empêche tout contact entre l'œuf O et la source lumineuse.

Le dispositif d'éclairage supérieur 6s et le dispositif d'éclairage inférieur 6i sont disposés en regard l'un de l'autre et dirigés l'un vers l'autre. Plus précisément, les lentilles 61 et les verres de protection 62 sont coaxiaux et les faisceaux lumineux émis par les diodes 60 sont orientés l'un vers l'autre et selon une même direction coaxiale à l'axe longitudinal des cavités 21, 41 et donc à l'axe longitudinal vertical de l'œuf O. Ainsi, le dispositif d'éclairage supérieur 6s est apte à émettre un faisceau lumineux qui pénètre dans l'œuf O depuis le dessus de l'œuf O et le dispositif d'éclairage inférieur 6i est apte à émettre un faisceau lumineux qui pénètre dans l'œuf O depuis le dessous de l'œuf O, de sorte que l'œuf O peut être éclairé depuis chacun de ses deux bouts.

Les moyens de connexion électrique 7 sont configurés pour relier électriquement chaque dispositif d'éclairage 6i, 6s à des moyens d'injection de courant 8 extérieurs à la navette 1. Ainsi, dans le mode de réalisation représenté, les moyens de connexion électrique 7 permettent de relier électriquement le dispositif d'éclairage supérieur 6s et le dispositif d'éclairage inférieur 6i à des moyens d'injection de courant 8.

Les moyens de connexion électrique 7 comprennent de manière générale un ensemble connecteur électrique 70 et un ensemble conducteur d'électricité formant un chemin électrique entre l'ensemble connecteur électrique 70 et chaque dispositif d'éclairage 6i, 6s.

Comme on peut le voir sur les Figures 1, 3 et 4, l'ensemble connecteur électrique 70 comprend ici trois connecteurs électriques fixés au socle 2. Ces trois connecteurs électriques 70 sont disposés l'un à côté de l'autre, parallèlement entre eux, et font saillie à partir d'une même face latérale du socle 2. En particulier, les trois connecteurs électriques 70 sont solidaires d'une face latérale située dans un plan vertical parallèle au plan comprenant les deux montants 3. Les trois connecteurs électriques 70 s'étendent perpendiculairement à cette face latérale verticale. Dans un mode de réalisation particulier de l'invention, les trois connecteurs électriques 70 sont des contacts à ressort.

L'ensemble conducteur d'électricité forme un chemin électrique entre l'ensemble connecteur 70 et le dispositif d'éclairage inférieur 6i. Comme on peut le voir sur la Figure 4, ce chemin comprend des éléments conducteurs 71, 72 intégrés dans le socle 2, en particulier dans l'espace intérieur du socle 2. Ces éléments conducteurs 71, 72 comprennent un fil électrique positif 71 reliant un connecteur électrique 70, notamment le connecteur central, et la borne positive de la diode 60 inférieure et un fil électrique négatif 72 reliant un autre connecteur électrique 70 et la borne négative de la diode 60 inférieure.

L'ensemble conducteur d'électricité forme également un chemin électrique entre l'ensemble connecteur 70 et le dispositif d'éclairage supérieur 6s. Comme on peut le voir sur les Figures 2 et 5, ce chemin comprend des éléments conducteurs 73, 74 intégrés dans le socle 2, des éléments conducteurs intégrés dans la bride 4 ainsi que les deux montants 3. Les éléments conducteurs 73, 74 intégrés dans le socle 2 comprennent un fil électrique positif 73 reliant un connecteur électrique 70, notamment le connecteur central, et l'un des deux montants verticaux 3 et un fil électrique négatif 74 reliant un autre connecteur 70 et l'autre montant vertical 3. Une cosse est fixée à l'extrémité inférieure de chaque montant 3 pour effectuer la connexion entre chaque montant 3 et le fil électrique associé. Les deux montants 3 sont réalisés en un matériau électroconducteur, de telle sorte qu'ils permettent la circulation d'un courant électrique entre l'extrémité inférieure et l'extrémité supérieure du montant 3. Les éléments conducteurs intégrés dans la bride 4 comprennent, de manière symétrique de part et d'autre de la diode 60 supérieure, un palier 75, une bride de fixation 76, une cosse 77 et un ressort de traction 78. Le palier 75 est un palier tubulaire en bronze monté autour du montant 3 associé. La bride de fixation 76 est une bride métallique serrée autour du palier 75 au moyen d'une vis 79 et d'un écrou. La cosse 77 est montée autour de la tige de la vis 79, entre deux écrous. Le ressort de traction 78 est également monté, à une extrémité, autour de la tige de la vis 79 et, à une autre extrémité, à un élément fixe solidaire de la bride, de façon à garantir un contact suffisant entre le palier 75 et le montant 3 respectif. Ainsi, la connexion électrique est assurée entre la bride de fixation 76 et chaque borne positive et négative de la diode 60. De ce fait, en utilisation, lorsque l'ensemble connecteur 70 est connecté à des moyens d'injection de courant 8, un courant peut circuler successivement dans le fil positif 73 intégré au socle 2, dans le montant 3 relié au fil positif 73, dans les éléments conducteurs 75-79 intégrés à la bride 4 en passant par la diode 60 supérieure, dans l'autre montant 3 puis dans le fil électrique négatif 74 intégré au socle 2.

En variante, les montants en matériau électroconducteur pourraient être remplacés par des montants en matériau isolant électrique traversés chacun par un fil électrique.

Une application préférée de la navette 1 selon la présente invention se situe dans le domaine avicole. Plus précisément, la navette 1 selon l'invention est particulièrement adaptée pour être utilisée pour le tri d'œufs O, par exemple d'œufs de cane, selon leur genre mâle ou femelle. Nous soulignons encore que la navette 1 selon l'invention est adaptée à d'autres articles séparés de forme ovoïde et pourrait être adaptée, en fonction de la forme et des dimensions des cavités supérieure 41 et inférieure 21, à tout autre article séparé. A cet effet, la navette 1 est destinée à être utilisée dans une machine M, notamment une machine M de tri automatique d'œufs O.

Dans un mode de réalisation préféré, une telle machine M adaptée au tri d'œufs O comprend : un poste de réception d'œufs, un poste de visiométrie, un poste de récupération d'œufs, une pluralité de navettes 1 telles que décrites ci-dessus, des moyens de transport 9 des navettes 1 et des moyens de préhension des œufs O. Nous soulignons que le nombre de navettes 1 peut être un nombre quelconque dépendant du nombre d'œufs O pouvant être saisis par les moyens de préhension. Plus le nombre de navettes 1 est élevé et plus la cadence de la machine M est élevée.

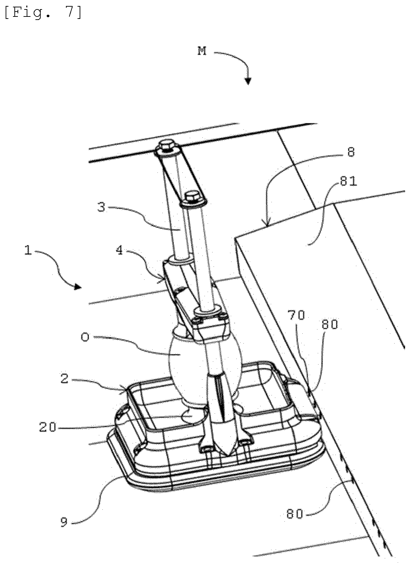

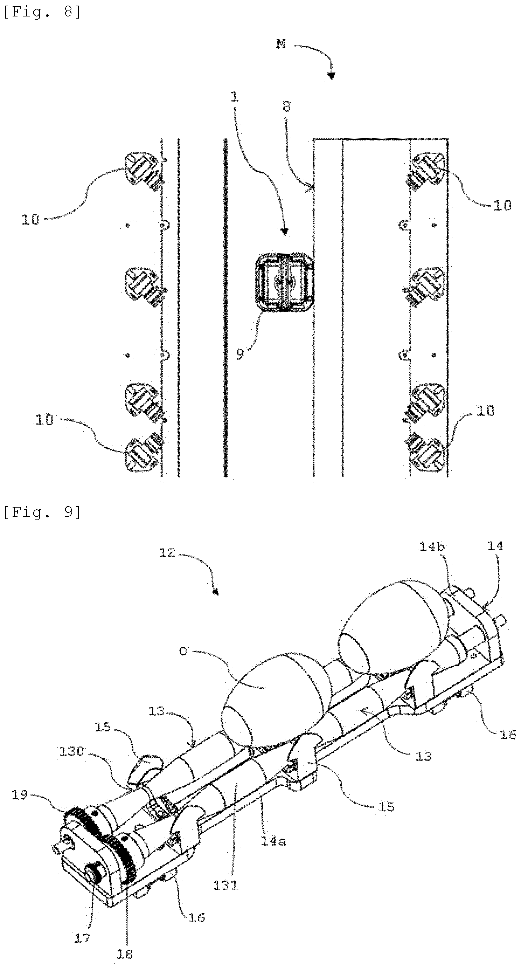

Si l'on se réfère désormais aux Figures 7 et 8, on peut voir que le poste de visiométrie comprend des moyens d'injection de courant 8 et une pluralité de caméras de prise de vues 10.

Les moyens d'injection de courant 8 sont aptes à alimenter en électricité une pluralité de navettes 1 accostées au poste de visiométrie. Les moyens d'injection de courant 8 comprennent une pluralité de séries de trois contacts d'alimentation électrique 80. Ces contacts d'alimentation 80 font saillie vers l'extérieur à partir d'un bloc d'alimentation 81 et sont disposés parallèlement entre eux le long d'un couloir longitudinal, l'axe des contacts 80 étant perpendiculaire à la direction longitudinale du couloir. Comme on peut le voir sur la Figure 7, les trois contacts d'alimentation 80 d'une même série sont agencés de façon à pouvoir se connecter aux trois connecteurs électriques 70 d'une navette 1. Les contacts d'alimentation électrique 80 des moyens d'injection de courant 8 sont des connecteurs complémentaires des connecteurs électriques 70 agencés sur la navette 1, les contacts d'alimentation électrique 80 et les connecteurs électriques 70 étant aptes à être mis en contact mécanique et électrique entre eux. Ainsi, dans le mode de réalisation particulier de l'invention décrit ci-dessus, les connecteurs électriques 70 agencés sur la navette 1 sont des contacts à ressort et les contacts d'alimentation électrique 80 sont des contacts complémentaires de ces contacts à ressort 70. L'agencement des contacts à ressort sur la navette 1 permet un démontage aisé de ces contacts lorsque leur remplacement est nécessaire. En variante, les contacts d'alimentation électrique 80 pourraient être des contacts à ressort et les connecteurs électriques 70 des connecteurs complémentaires de ces contacts à ressort. L'agencement des contacts à ressort au niveau des moyens d'injection de courant 8, plutôt que sur la navette 1, permet de faciliter une identification des pannes.

On entend par le terme « caméra » tout dispositif de prise de vues, donc comprenant aussi bien une caméra vidéo qu'un appareil photo numérique. Comme on peut le voir sur la Figure 8, les caméras 10 sont positionnées de part et d'autre du couloir longitudinal. La position en hauteur des caméras 10 est définie de telle sorte qu'elles sont aptes à capturer des images de la surface de l'œuf O laissée libre lorsque l'œuf O est positionnée sur la navette 1. Les caméras 10 sont orientées dans un plan horizontal de telle sorte qu'un œuf O positionné sur une navette 1 peut être photographié par quatre caméras 10, à savoir une première paire de caméras 10 d'un premier côté du couloir et une seconde paire de caméras 10 d'un second côté du couloir. De manière connue en soi, chaque caméra 10 est associée à des moyens d'analyse automatique (non représentés) aptes à analyser les images générées par chaque caméra 10, notamment aptes à déterminer le genre de l'œuf O à partir des images des caméras 10.

Ainsi, en utilisation, une fois l'œuf O maintenu en position sur la navette 1, la navette 1 est amenée au poste de visiométrie jusqu'à une position d'accostage, dans laquelle la navette 1 est reliée aux moyens d'injection de courant 8. La navette 1 marque alors un temps d'arrêt à cette position d'accostage. Pendant ce temps d'arrêt, l'alimentation électrique des dispositifs d'éclairage 6i, 6s est commandée afin d'éclairer l'œuf O de manière suffisante et optimale. En particulier, le dispositif d'éclairage supérieur 6s est alimenté de manière à émettre de la lumière, par exemple sous la forme d'un faisceau lumineux, depuis le dessus de l'œuf O, l'œuf O éclairé par le dessus étant alors photographié par les caméras 10. Ensuite, le dispositif d'éclairage inférieur 6i est alimenté de manière à émettre de la lumière, par exemple sous la forme d'un faisceau lumineux, depuis le dessous de l'œuf O, l'œuf O éclairé par le dessous étant alors photographié par les caméras 10. Une fois que l'ensemble des images souhaitées sont captées par les caméras 10, la navette 1 est à nouveau déplacée vers le poste suivant, à savoir le poste de récupération d'œufs. Au niveau du poste de récupération d'œufs, en fonction du résultat de l'analyse des images de l'œuf éclairé, chaque navette 1 vient se positionner à un emplacement prédéterminé. Par exemple, les navettes 1 transportant un œuf mâle se placent d'un côté et les navettes 1 transportant un œuf femelle se placent d'un autre côté. Ainsi, dans cette application particulière de la machine, les navettes peuvent être rangées au niveau du poste de récupération d'œufs dans un ordre différent de leur ordre de départ au niveau du poste de réception d'œufs, et ceci en fonction du sexe de l'œuf présenté par chaque navette.

Ensuite, un ensemble de préhension aval (non représenté) vient soulever les brides 4 des navettes 1 ainsi triées et récupère les œufs O afin de les transférer dans un casier à œufs (non représenté) dans une position appropriée. Les œufs mâles peuvent être transférés dans un casier à œufs mâles et les œufs femelles dans un casier à œufs femelles. Plusieurs navettes 1 peuvent être déplacées en même temps, les unes à la suite des autres, et photographiées de manière simultanée par la pluralité de caméras 10. Ainsi, une pluralité d'œufs O peuvent être observés en même temps et triés à une cadence élevée. La possibilité d'éclairer l'œuf O depuis le dessus de l'œuf et depuis le dessous de l'œuf permet de faciliter l'analyse des images par les moyens d'analyse, et donc de parvenir à un tri des œufs O fiable et rapide.

Comme on peut le voir sur la Figure 7, les moyens de transport 9 de chaque navette 1, permettant son déplacement du poste de réception au poste de visiométrie, le long du couloir du poste de visiométrie, puis jusqu'au poste de récupération, comprennent de préférence un moteur plan à sustentation magnétique 9 disposé au-dessous du socle 2 de la navette 1. Ce moteur plan 9 peut avoir sensiblement les dimensions de la face inférieure du socle 2. Un tel moteur plan 9 est connu en soi et permet un déplacement des navettes 1 sans fil et sans batterie.

Afin d'assurer un positionnement correct de chaque œuf O sur sa navette 1, le poste de réception d'œufs comprend des moyens permettant d'orienter et d'aligner des œufs O issus d'un casier à œufs classique. Ainsi, les œufs O sont transférés sur les navettes 1 par un ensemble de préhension amont (non représenté) après leur passage par ces moyens d'orientation et d'alignement.

Si l'on se réfère désormais aux Figures 9 à 15, on peut voir que ces moyens d'orientation et d'alignement comprennent au moins un plateau 11 d'orientation et d'alignement d'œufs, de préférence deux plateaux 11, chaque plateau 11 comprenant au moins un module 12 d'orientation et d'alignement d'œufs, de préférence une pluralité de modules 12.

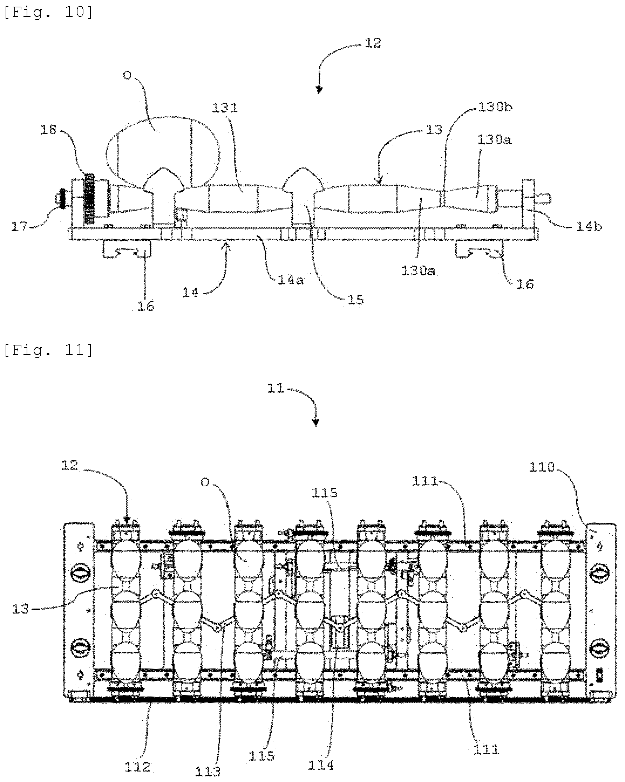

Comme on peut le voir sur les Figures 9 et 10, chaque module 12 comprend une paire de rouleaux 13 montés rotatifs sur un support en U 14.

Le support en U 14 comprend une plaque principale 14a et deux ailes latérales 14b. Les deux ailes latérales 14b sont agencées en regard l'une de l'autre, à chaque extrémité longitudinale de la plaque principale 14a, dans des plans parallèles entre eux, et perpendiculairement au plan de la plaque principale 14a. Chaque aile latérale 14b comporte une paire d'orifices juxtaposés formés à travers l'aile 14b. Les axes des orifices sont parallèles entre eux et parallèles à la direction longitudinale de la plaque principale 14a. La paire d'orifices de l'une des ailes 14b se trouve en regard de la paire d'orifices de l'aile 14b opposée. Les orifices sont dimensionnés pour recevoir une région d'extrémité des rouleaux 13. Les orifices d'une même paire sont espacés l'un de l'autre d'une certaine distance transversale.

La paire de rouleaux 13 comprend un premier rouleau et un second rouleau. Le premier rouleau est reçu à travers deux orifices en regard des ailes 14b et le second rouleau est reçu à travers deux autres orifices en regard des ailes 14b. Ainsi, les deux rouleaux 13 s'étendent selon la direction longitudinale de la plaque principale 14a et leurs axes sont parallèles entre eux. Un palier lisse peut être logé à l'intérieur de chaque orifice, entre la paroi de l'orifice et le rouleau 13 associé. La longueur de chaque rouleau 13 est supérieure à la longueur du support 14, de telle sorte que chaque rouleau 13 fait saillie de part et d'autre du support 14, au-delà de chaque aile 14b. L'espacement entre les rouleaux 13 est tel que des œufs O peuvent être reçus à l'horizontale, autrement dit avec leur axe longitudinal parallèle à l'axe de chaque rouleau 13, sur et entre les deux rouleaux 13. Chaque rouleau 13 présente plusieurs sections de différents diamètres. En particulier, ici chaque rouleau 13 a trois sections de calage 130 et une section cylindrique 131 entre la section de calage 130 centrale et chaque section de calage 130 d'extrémité. Chaque section de calage 130 a une forme générale de diabolo, c'est-à-dire qu'elle est essentiellement constituée de deux portions tronconiques 130a coaxiales allant en rétrécissant l'une vers l'autre et reliées dans leur zone de petit diamètre par une portion cylindrique 130b). Ainsi, chaque section de calage 130 présente une portion cylindrique 130b médiane étroite constituant une gorge et des extrémités élargies. Les sections de calage 130 des deux rouleaux 13 sont juxtaposées par paires. Autrement dit, chaque gorge 130b de l'un des rouleaux 13 se trouve dans le même plan transversal qu'une gorge 130b de l'autre rouleau 13. Ces sections de calage 130 permettent chacune le calage longitudinal d'un œuf O sur le module 12. Il convient de souligner que le nombre de sections de calage 130 peut être variable, en fonction du nombre d'œufs O que l'on souhaite traiter de manière simultanée.

Pour permettre le calage transversal de chaque œuf O sur le module 12, une paire de guides extérieurs 15 est prévue de chaque côté de la paire de rouleaux 13 au niveau de chaque gorge 130b. Chaque guide extérieur 15 est solidaire de la plaque principale 14a et fait saillie au-dessus des rouleaux 13. Deux guide extérieurs 15 d'une même paire sont disposés en regard l'un de l'autre. La distance transversale entre deux guides extérieurs 15 d'une même paire est légèrement supérieure au diamètre maximal des œufs O de façon à empêcher toute chute des œufs O reçus dans les gorges 130b.

Pour permettre le montage à coulissement du module 12, selon une direction transversale, donc dans un plan horizontal et perpendiculairement aux axes des rouleaux 13, le module 12 comporte une paire de patins 16. Les patins 16 sont fixés à la face inférieure de la plaque principale 14a.

Pour permettre aux œufs O positionnés sur le module 12 de s'orienter automatiquement à l'horizontale et avec leurs petits bouts orientés dans un même sens, le module 12 porte des moyens de mise en contre-rotation des rouleaux 13 de la paire de rouleaux 13. Ces moyens comprennent un jeu de pignons 17-19, dont un premier pignon 17, un pignon intermédiaire 18 et un second pignon 19. Le premier pignon 17 est solidaire du premier rouleau 13 et est monté autour d'une région d'extrémité du premier rouleau 13 qui fait saillie à l'extérieur du support 14. Autrement dit, le premier pignon 17 est positionné au voisinage de la face extérieure de l'aile 14b correspondante. Le pignon intermédiaire 18 est également solidaire du premier rouleau 13. Le pignon intermédiaire 18 est monté autour du premier rouleau 13, ici entre la face intérieure de l'une des ailes 14b et la section de calage 130 d'extrémité correspondante. Le second pignon 19 est solidaire du second rouleau 13 et est juxtaposé au pignon intermédiaire 18. Les diamètres du second pignon 19 et du pignon intermédiaire 18 sont tels que la denture portée par le second pignon 19 s'engrène sur la denture portée par le pignon intermédiaire 18. Ainsi, une rotation du premier pignon 17 dans un premier sens de rotation entraîne la rotation du pignon intermédiaire 18 et donc du premier rouleau 13 dans ce premier sens de rotation, et la rotation du pignon intermédiaire 18 dans le premier sens de rotation entraîne à son tour la rotation du second pignon 19 et donc du second rouleau 13 dans un second sens de rotation, opposé au premier sens de rotation.

Comme on peut le voir sur les Figures 11 à 13, le ou chaque plateau 11 d'orientation et d'alignement d'œufs selon la présente invention comprend une pluralité de modules 12, par exemple huit modules 12 tels que décrits ci-dessus. Un tel plateau 11 à huit modules 12 ayant chacun trois sections de calage 130 peut traiter 24 œufs à la fois.

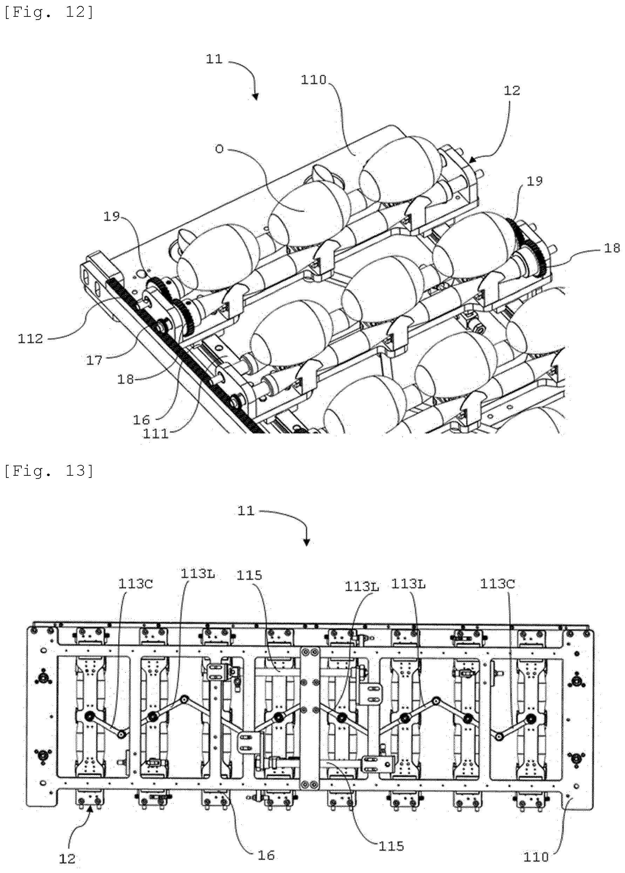

Comme on peut le voir sur les Figures 11 et 12, les modules 12 sont supportés par un cadre de support 110 comprenant deux rails de guidage 111 configurés pour coopérer à coulissement avec les patins 16. Les deux rails 111 s'étendent dans un même plan horizontal parallèlement entre eux et sont espacés l'un de l'autre d'une distance correspondant à la distance entre les deux patins 16 d'une même paire. Le profil extérieur de chaque rail 111 correspond au profil intérieur de chaque patin 16. Ainsi, les modules 12 sont montés mobiles en translation le long des rails de guidage 111 dans une direction qui est parallèle à la direction longitudinale du cadre de support 110 et perpendiculaire à la direction longitudinale de chaque module 12.

A titre d'exemple, le cadre de support 110 peut avoir une longueur de l'ordre de 1 mètre et une largeur de l'ordre de 30 à 35 centimètres.

Comme on peut également le voir sur les Figures 11 et 12, le cadre de support 110 comprend également une crémaillère 112 apte à coopérer avec le jeu de pignons 17-19 pour l'entraînement en contre-rotation des rouleaux 13. Plus précisément, la crémaillère 112 est disposée d'un côté du plateau 11 selon une direction longitudinale parallèle aux rails de guidage 111 de façon à pouvoir venir en prise avec le premier pignon 17 de chaque module 12. Ainsi, un déplacement du module 12 dans la direction longitudinale de la crémaillère 112 entraîne la mise en contre-rotation des rouleaux 13 de ce module 12.

Afin d'éviter tout risque de contact entre le second pignon 19 de l'un des modules 12 et le pignon intermédiaire 18 d'un module 12 adjacent, en particulier dans le cas où les modules 12 sont positionnés de telle sorte que les supports 14 de deux modules 12 adjacents viennent en contact entre eux, le pignon intermédiaire 18 et le second pignon 19 d'un module 12 sont agencés à une région d'extrémité longitudinale du module 12 opposée à celle à laquelle sont agencés le pignon intermédiaire 18 et le second pignon 19 d'un module 12 adjacent.

Comme on peut le voir sur les Figures 11 et 13, le cadre de support 110 porte également des moyens de commande du déplacement en translation des modules 12 le long des rails de guidage 111. Ces moyens sont un système de mise au pas des modules configuré pour permettre le déplacement en translation de l'ensemble des modules 12 de manière simultanée et avec un pas constant entre les modules 12. Le système de mise au pas comprend des bielles articulées 113, un support de guidage 114 et deux vérins 115. Les bielles articulées 113 relient entre eux les modules 12 et sont articulées autour d'axes verticaux, autrement dit autour d'axes perpendiculaires au plan de déplacement en translation des modules 12. Dans le mode de réalisation représenté, dans lequel le plateau 11 comprend huit modules 12, les bielles articulées 113 comprennent une bielle courte 113C reliée de manière pivotante à chaque module 12 d'extrémité et six bielles longues 113L articulées autour d'axes de pivot verticaux et entre les bielles courtes 113C auxquelles elles sont reliées de manière pivotante. Chaque bielle longue 113L est reliée de manière pivotante à l'un des modules 12. L'axe de pivot entre chaque bielle 113 et le module 12 associé s'étend à partir de la face inférieure de la plaque principale 14a, au centre de la plaque principale 14a dans le sens de sa longueur, et perpendiculairement à cette plaque. L'axe de pivot entre chaque bielle courte 113C et le module 12 associé traverse la bielle courte 113C au niveau de l'une de ses extrémités. L'axe de pivot entre chaque bielle longue 113L et le module 12 associé traverse la bielle longue 113L en son centre. Chaque axe de pivot peut comprendre une vis épaulée et deux paliers lisses à collerette de part et d'autre de la bielle. Les articulations entre les bielles 113 et entre chaque bielle 113 et l'un des modules 12, conjointement au montage coulissant des modules 12, donnent à l'ensemble de bielles 113 une forme globale de pantographe, pouvant se replier et se déplier à la manière d'un accordéon.

Le support de guidage 114 est solidaire du cadre de support 110. Ce support de guidage 114 comprend un guide 114 horizontal à section transversale en U s'étendant orthogonalement aux rails 111. Ce guide 114 s'étend au-dessus des bielles 113, entre les deux modules 12 centraux. L'axe d'articulation entre les deux bielles longues 113L centrales est reçu dans ce guide horizontal 114, permettant ainsi de guider le déplacement en translation de l'axe central selon une direction parallèle aux rouleaux 13.

Les deux vérins 115 sont les moyens d'actionnement permettant de commander le déplacement des bielles articulées 113 et donc de l'ensemble des modules 12 reliés entre eux par les bielles 113. Les deux vérins 115 s'étendent dans un plan horizontal situé au-dessous du plan des modules 12. Les vérins 115 ont des axes parallèles entre eux et parallèles aux rails de guidage 111. Chaque vérin 115 est agencé entre un rail de guidage 111 et les bielles articulées 113. Chaque vérin 115 a une tige dont la tête est reliée à une chape portée par la face inférieure de la plaque principale 14a de l'un des modules 12. En particulier, la tête de l'un des vérins 115 est reliée au module 12 adjacent à l'un des modules d'extrémité et la tête de l'autre vérin 115 est reliée au module 12 adjacent à l'autre module d'extrémité. Ainsi, les deux vérins 115 sont montés dans des sens opposés.

En utilisation, des œufs O sont prélevés depuis un casier à œufs, notamment un casier en fil d'acier, au moyen d'outils de saisie (non représentés), notamment de ventouses saisissant les œufs O par le côté. Dans le casier, les œufs O ne sont pas calibrés et ont une position quelconque. Ces œufs O sont ensuite déposés couchés sur le plateau 11 selon la présente invention, en particulier dans les sections de calage 130 de chaque module 12. Dans le cas d'un plateau 11 à huit modules 12, les œufs O peuvent être transférés par un ensemble comprenant huit outils de saisie, notamment huit ventouses, alignés et espacés d'un certain pas, lequel pas correspond au pas entre les œufs O d'une même rangée dans le casier. Dans ce cas, avant le dépôt sur le plateau 11 de la rangée de huit œufs O prélevés depuis le casier, le système de mise au pas des modules est actionné de telle sorte que le pas des modules 12 est égal au pas des œufs O dans le casier. Ainsi, les œufs O peuvent être déposés facilement et correctement sur le plateau 11, chaque œuf O venant se positionner dans une section de calage 130 de l'un des modules 12. La mise en contre-rotation des rouleaux 13 de chaque module 12 amène alors chaque œuf O à se positionner avec son axe longitudinal à l'horizontal et son petit bout vers l'un des côtés du plateau 11. L'ensemble des œufs se trouvent alors dans la même position. Ce positionnement correct et identique des œufs O sur le plateau 11 permet par la suite à l'ensemble de préhension amont de positionner correctement les œufs O issus du plateau 11 sur les navettes 1, et contribue donc à une présentation optimale des œufs O transportés par les navettes 1. Cette présentation optimale permet une prise de photos de bonne qualité au niveau du poste de visiométrie, et donc un tri fiable et rapide des œufs O.

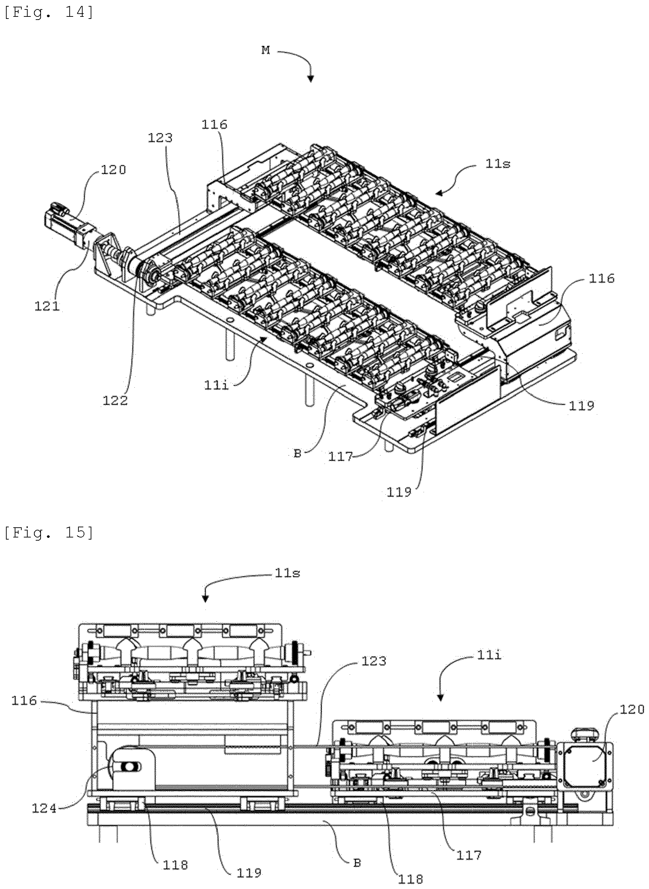

Comme on peut le voir sur les Figures 14 et 15, le poste de réception d'œufs comprend, de préférence, deux plateaux 11 tels que décrits ci-dessus.

Les deux plateaux 11 sont supportés sur un bâti de support B. L'un des plateaux, dit plateau supérieur 11s, est supporté à une hauteur supérieure à l'autre plateau, dit plateau inférieur 11i. La différence de hauteur entre les deux plateaux 11s, 11i est telle que le plateau supérieur 11s peut passer au-dessus du plateau inférieur 11i. Pour cela, le cadre de support 110 du plateau supérieur 11s est fixé, à chacune de ses deux extrémités longitudinales, à un support latéral 116 ayant globalement une forme en L. le cadre de support 110 du plateau inférieur 11i est fixé, à chacune de ses deux extrémités longitudinales, à un support latéral plan 117. Chaque support latéral 116, 117 est porté par deux coulisseaux 118. Le bâti de support B comprend quatre rails 119, dont deux rails 119 le long desquels les coulisseaux 118 du plateau inférieur 11i sont aptes à coulisser et deux rails 119 le long desquels les coulisseaux 118 du plateau supérieur 11s sont aptes à coulisser. Les rails 119 s'étendent sur sensiblement toute la largeur du bâti de support B, dans un plan horizontal, parallèlement entre eux et perpendiculairement à la direction longitudinale des plateaux 11. Ainsi, les plateaux 11i, 11s sont aptes à se déplacer en translation horizontale selon une direction orthogonale à la direction de translation des modules 12 par rapport au cadre de support 110.

Le déplacement en translation des deux plateaux 11i, 11s le long des rails transversaux 119 et l'un par rapport à l'autre est commandé un unique moteur 120 et des moyens de transmission par poulie et courroie portés par le bâti de support B. Le moteur 120 a un arbre moteur qui est accouplé, par l'intermédiaire d'un réducteur 121, à une poulie menante 122. L'arbre moteur s'étend selon un axe horizontal orthogonal aux quatre rails 119. Une courroie sans fin 123 coopère avec la poulie menante 122 et est tendue entre la poulie menante 122 et une poulie menée 124. Les poulies menante 122 et menée 124 sont agencées sur un côté du bâti B, entre deux rails transversaux 119. Le brin inférieur de la courroie 123 est solidaire du support latéral 117 coulissant correspondant du plateau inférieur 11i, par l'intermédiaire d'un organe de jonction. Le brin supérieur de la courroie 123 est solidaire du support latéral 116 coulissant correspondant du plateau supérieur 11s, par l'intermédiaire d'un autre organe de jonction analogue. Ainsi, lorsque l'un des plateau se déplace dans une direction transversale, l'autre plateau se déplace dans la direction transversale opposée. Les organes de jonction sont agencés de telle sorte que le plateau inférieur 11i et le plateau supérieur 11s peuvent être positionnés l'un à côté de l'autre en vue de dessus.

Un tel système comprenant deux plateaux 11s, 11i mobiles transversalement de manière synchrone permet de placer des œufs O sur l'un des plateaux tout en prélevant des œufs O depuis l'autre plateau, et permet donc d'augmenter la cadence de la machine M.

Il est bien entendu que les modes de réalisation ci-dessus de la présente invention ont été donnés à titre indicatif et non limitatif et que des modifications pourront y être apportées sans que l'on s'écarte pour autant du cadre de la présente invention.

## Revendications

1. - Navette (1) de support et d'éclairage d'au moins un objet, notamment un objet à trier tel qu'un œuf aviaire (O), destinée à être positionnée ou déplacée dans un poste de visiométrie ou d'inspection optique, laquelle navette (1) comporte un ensemble de maintien (2, 3, 4) configuré pour supporter et maintenir en position l'au moins un objet, et un système d'éclairage (6) configuré pour éclairer l'au moins un objet, la navette (1) étant **caractérisée par le fait que** :
- l'ensemble de maintien (2, 3, 4) comprend un socle (2) et une bride (4) supportée par le socle (2), le socle (2) comporte au moins un siège (20) définissant une cavité inférieure (21) agencée pour recevoir une région d'extrémité inférieure d'un objet et ayant une ouverture de réception inférieure (22) apte à coopérer avec l'objet, la bride (4) comporte, en regard du ou de chaque siège (20), une cavité supérieure (41) agencée pour recevoir une région d'extrémité supérieure de l'objet et ayant une ouverture de réception supérieure (42) apte à coopérer avec l'objet, l'ouverture de réception inférieure (22) et l'ouverture de réception supérieure (42) associée étant orientées l'une vers l'autre et selon un même axe, en utilisation, l'ensemble de maintien (2, 3, 4) étant configuré pour être apte à occuper une position écartée, dans laquelle la ou chaque ouverture de réception inférieure (22) et l'ouverture de réception supérieure (42) associée sont espacées l'une de l'autre d'une distance autorisant le dépôt d'un objet sur le siège (20) et son retrait, et une position de maintien, dans laquelle la ou chaque ouverture de réception inférieure (22) et l'ouverture de réception supérieure (42) associée sont rapprochées l'une de l'autre d'une distance permettant le maintien et l'immobilisation d'un objet entre le socle (2) et la bride (4), avec les régions d'extrémité inférieure et supérieure de l'objet reçues dans la cavité inférieure (21) et dans la cavité supérieure (41), respectivement ;
- le système d'éclairage (6) comprend, dans au moins l'une des cavités inférieure (21) et supérieure (41), un dispositif d'éclairage (6s, 6i) configuré pour émettre de la lumière en direction de l'ouverture de réception (22, 42) associée ; et
- la navette (1) comprenant en outre des moyens de connexion électrique (7) comportant un ensemble connecteur électrique (70) accessible depuis l'extérieur du socle (2) et un ensemble conducteur d'électricité (71-79) formant un chemin électrique entre l'ensemble connecteur électrique (70) et le ou chaque dispositif d'éclairage (6i, 6s), de telle sorte qu'en utilisation, lorsque l'ensemble connecteur (70) est relié à des moyens d'injection courant (8), le ou chaque dispositif d'éclairage (6i, 6s) est apte à être alimenté en électricité.

2. - Navette (1) selon la revendication 1, **caractérisée par le fait que** la bride (4) est montée mobile à coulissement libre vertical par rapport au socle (2), le socle (2) portant au moins un guide vertical (3) le long duquel la bride (4) est apte à coulisser.

3. - Navette (1) selon la revendication 2, **caractérisée par le fait que**, pour le ou chaque dispositif d'éclairage supérieur (6s), le chemin électrique comprend des fils électriques (73, 74) intégrés au socle (2) et reliant l'ensemble connecteur électrique (70) à l'extrémité inférieure du ou de chaque guide vertical (3), le ou chaque guide vertical (3) réalisé en un matériau électroconducteur et une pluralité d'éléments conducteurs (75-79) intégrés à la bride (4) et reliant le ou de chaque guide vertical (3) et le dispositif d'éclairage supérieur (6s), et **par le fait que**, pour le ou chaque dispositif d'éclairage inférieur (6i), le chemin électrique comprend des fils électriques (71, 72) intégrés au socle (2) et reliant directement l'ensemble connecteur électrique (70) et le dispositif d'éclairage inférieur (6i).

4. - Navette (1) selon l'une quelconque des revendications 1 à 3, **caractérisée par le fait que** le système d'éclairage (6) comprend un dispositif d'éclairage supérieur (6s) dans la ou chaque cavité supérieure (41) et un dispositif d'éclairage inférieur (6i) dans la ou chaque cavité inférieure (21), chaque dispositif d'éclairage supérieur (6s) et le dispositif d'éclairage inférieur (6i) associé étant avantageusement agencés pour émettre des faisceaux lumineux passant par l'axe commun des ouvertures de réception (42, 22) associées.

5. - Machine (M), notamment machine de tri automatique d'objets, ladite machine (M) étant **caractérisée par le fait qu'**elle comprend au moins une navette (1) de support et d'éclairage selon l'une quelconque des revendications 1 à 4, la machine (M) comprenant en outre un poste de réception d'objets, un poste de visiométrie et un poste de récupération d'objets, des moyens de transport (9) pour déplacer l'au moins une navette (1) selon un trajet passant par les divers postes, un ensemble de préhension amont apte à saisir au moins un objet au poste de réception et à le positionner dans l'au moins une navette (1), et un ensemble de préhension aval apte à saisir au moins un objet dans l'au moins une navette (1) et à le transférer au poste de récupération, le poste de visiométrie comprenant une pluralité de caméras de prise de vues (10) associées à des moyens d'analyse automatique et des moyens d'injection de courant (8) aptes à être connectés à l'ensemble connecteur électrique (70) de l'au moins une navette (1).

6. - Machine (M) selon la revendication 5, **caractérisée par le fait que** les moyens de transport (9) comprennent un moteur plan à sustentation magnétique (9) sur lequel la ou les navettes (1) sont disposées.

7. - Machine (M) selon l'une quelconque des revendications 5 et 6, **caractérisée par le fait qu'**elle est adaptée au tri d'œufs (O), le poste de réception comprenant au moins un plateau (11) d'orientation et d'alignement d'œufs, le ou chaque plateau (11) comprenant au moins un module (12) d'orientation et d'alignement, le ou chaque module (12) comprenant deux rouleaux (13) rotatifs axialement parallèles et espacés l'un de l'autre d'une distance permettant le positionnement d'un œuf (O) sur et entre les deux rouleaux (13), et un mécanisme (17-19, 112) d'entraînement en rotation configuré pour entraîner les deux rouleaux (13) d'un même module (12) en contre-rotation l'un par rapport à l'autre et vers l'espace inter-rouleaux.

8. - Machine (M) selon la revendication 7, **caractérisée par le fait que** le ou chaque plateau (11) comprend une pluralité de modules (12) d'orientation et d'alignement dont les rouleaux (13) sont agencés parallèlement les uns aux autres, les modules (12) sont montés mobiles en translation le long de deux rails de guidage (111) parallèles entre eux et dont les axes perpendiculaires aux axes des rouleaux (13), par l'intermédiaire de patins (16) montés coulissants le long des rails (111), chaque module (12) étant supporté par deux patins (16) solidaires de chaque extrémité longitudinale du module (12).

9. - Machine (M) selon la revendication 8, **caractérisée par le fait que** le ou chaque plateau (11) comprend un système de mise au pas des modules configuré pour piloter le déplacement en translation de la pluralité de modules (12) le long des rails de guidage (111) avec un pas constant entre les modules (12), lequel système de mise au pas comprend une pluralité de bielles (113) articulées entre elles autour d'axes perpendiculaires au plan de déplacement en translation des modules (12), chaque bielle (113) étant solidaire d'un module (12), l'axe d'articulation entre deux bielles (113) dites bielles centrales étant reçu dans un moyen de guidage (114) en translation suivant une direction de translation perpendiculaire à l'axe d'articulation et parallèle aux axes des rouleaux (13), deux vérins d'actionnement (115) disposés de part et d'autre de la pluralité de bielles (113), parallèlement entre eux et orthogonalement aux axes des rouleaux (13) et aux axes d'articulation, étant solidaires de deux modules (12), de telle sorte qu'un déplacement en translation relatif entre les deux modules (12) d'un pas prédéterminé, sous l'effet des vérins (115), provoque un déplacement en translation synchrone de chaque module (12) avec un pas entre modules (12) égal audit pas prédéterminé.

10. - Machine (M) selon l'une quelconque des revendications 7 à 9, **caractérisée par le fait que** le ou chaque plateau (11) est monté mobile en translation le long de deux rails transversaux (119) de guidage, les rails transversaux (119) étant parallèles entre eux et parallèles aux axes des rouleaux (13), chaque extrémité longitudinale du ou de chaque plateau (11) étant solidaire d'un support latéral (116, 117) monté coulissant le long d'un rail (119) respectif, l'un des deux support (116, 117) du ou de chaque plateau (11) étant solidaire d'une courroie synchrone (123) tendue entre deux poulies (122, 124), l'une des poulies (122) étant accouplée à un motoréducteur (120-121).
